# EUROPEAN PATENT APPLICATION

(11) **EP 4 364 638 A1**
(43) Date of publication of application: **08.05.2024**
(21) Application number: 22831747.5
(22) Date of filing: 20.06.2022
(51) Int. Cl.: A61B 1/12, A61B 1/04

(54) **FLUID-ASSISTED MINIMALLY INVASIVE SURGERY VISUALIZATION DEVICE**

(30) Priority: 30.06.2021 CN 202110732593; 03.12.2021 CN 202111465148
(71) Applicant: Li, Guangcheng, Qingdao, Shandong 266400 (CN)
(72) Inventor: LIU, Jian, Qingdao, Shandong 266400 (CN); ZHANG, Wenyong, Qingdao, Shandong 266400 (CN); LI, Guangcheng, Qingdao, Shandong 266400 (CN)
(74) Representative: Bandpay & Greuter
(86) International application number: PCT/CN2022/099662
(87) International publication number: WO 2023/273929

(57) **Abstract**

The present disclosure provides a fluid-assisted minimally-invasive surgery visualization device, which includes an outer sleeve assembly, an imaging module, an imaging module support body and a water delivery pipe. The outer sleeve assembly includes an outer sleeve and a sleeve seat. A water supply cavity in communication with a rear end of the outer sleeve is provided inside the sleeve seat, and the water supply cavity is further in communication with the water delivery pipe. The imaging module is supported on the outer sleeve assembly by the imaging module support body, the imaging module is accommodated in the outer sleeve, and one segment of clear water cavity is formed between a front end surface of the outer sleeve and a lens of the imaging module. In the present disclosure, a clear water region capable of providing clear view is present between the lens and the front end surface of the outer sleeve, and the clear water flowing forward inside the outer sleeve flushes away bloody water to form a clear visual region, thereby improving the accuracy and efficiency of the surgical operation.

## Description

### TECHNICAL FIELD

The present disclosure relates to the field of medical appliance technologies and in particular to a fluid-assisted minimally-invasive surgery visualization device.

### BACKGROUND

In the current clinical surgical applications, an endoscope (e.g. ventriculoscope) under liquid phase must be used in a clear liquid environment. When obvious bleeding occurs, bloody water before a lens of the endoscope will block a view of the lens and a blurred region is formed between the lens and a tissue surface, making it difficult to clearly observe an injury region and a bleeding point of the tissue surface. The main reason is that, although the existing endoscope has a flushing water port and a return water pipe mouth disposed on a front end surface of an outer sleeve, the lens is mounted at the front end surface of the water sleeve, namely, mounted in an open space; after a clear normal saline is injected through a water outlet end, the normal saline will immediately mix with the bloody water to form a bloody turbid liquid, unable to form a clear view region before the lens. As a result, it is impossible to accurately determine a lesion site by the lens, severely affecting the accuracy of the surgical operation, reducing the surgical efficiency and resulting in high possibility of occurrence of mis-operation. Therefore, improvements are to be made on the prior arts.

### SUMMARY

For the defects existing in the prior arts, the present disclosure provides a fluid-assisted minimally-invasive surgery visualization device, so as to solve the problems of improper lens position of the existing endoscope operation devices and difficulty in forming a clear visual region before the lens in a bloody environment.

For the above objects, the present disclosure employs the following technical solution.

There is provided a fluid-assisted minimally-invasive surgery visualization device, including an outer sleeve assembly, an imaging module, an imaging module support body and a water delivery pipe. The outer sleeve assembly includes an outer sleeve and a sleeve seat; a water supply cavity in communication with a rear end of the outer sleeve is provided inside the sleeve seat; the water supply cavity is further in communication with the water delivery pipe; the imaging module is supported on the outer sleeve assembly by the imaging module support body, the imaging module is accommodated in the outer sleeve, and one segment of clear water cavity is formed between a front end surface of the outer sleeve and a lens of the imaging module.

Preferably, the outer sleeve and the sleeve seat are of integral structure or of split structure.

Preferably, the imaging module support body is supported on the outer sleeve or on the sleeve seat.

Preferably, the imaging module support body is a rod body, or a support bracket, or a hard wire body, or a cooperative structure of rod body and support bracket.

Preferably, the imaging module support body is a rod body; a rear end of the rod body is supported on a rear part of the sleeve seat; a first through hole is disposed at a part of the sleeve seat located behind the water supply cavity, and the rod body is supported and mounted at the rear part of the sleeve seat by the first through hole.

Preferably, all or part of the outer sleeve is a flexible pipe.

Preferably, a water supply hole in communication with the water supply cavity is disposed on a wall of the sleeve seat; and the water supply hole is connected with the water delivery pipe.

Preferably, a front end part of the outer sleeve is bent aside to form a first bending portion, and a front end part of the imaging module support body is bent aside to form a second bending portion; the second bending portion is consistent in direction with the first bending portion, and the clear water cavity is located inside the first bending portion.

Preferably, the front end of the outer sleeve is provided with a connection portion used for connecting an operation tool.

Preferably, the connection portion is internal threads for connecting the operation tool, which are disposed on an inner sidewall of the front end of the outer sleeve, and located ahead of the lens.

Preferably, the rear end of the sleeve seat is further provided with one or more operation holes, and the operation hole forms a working channel communicating the interior of the outer sleeve assembly with the outside.

Preferably, the device includes an inner pipe body. The inner pipe body is disposed at the operation hole, a working channel communicating the interior of the outer sleeve with the outside is formed inside the inner pipe body, and the number of the inner pipe bodies is equal to or less than the number of the operation holes.

Preferably, a front end surface of the inner pipe body is located behind the lens.

Preferably, a front end of the inner pipe body is provided with an elastic tightening portion with an inner diameter less than an inner diameter of the inner pipe body.

Preferably, the device includes a sealing cap for sealing the operation hole.

By using the above technical solution, the present disclosure has the following beneficial technical effects.

In the present disclosure, the lens of the imaging module is located inside the outer sleeve, and one segment of clear water is maintained between the lens and the front end surface of the outer sleeve. Clear water flowing forward inside the outer sleeve forms a clear visual region. When the front end of the outer sleeve approaches a tissue surface, the clear water inside it flushes away the bloody water to make the tissue surface clearly observable, thereby improving the accuracy and efficiency of the surgical operation and eliminating the possibility of mis-operation.

### BRIEF DESCRIPTIONS OF THE DRAWINGS

FIG. 1 is a structural schematic diagram illustrating a first implementation of a fluid-assisted minimally-invasive surgery visualization device according to the present disclosure.
FIG. 2 is a structural sectional diagram illustrating a first implementation of a fluid-assisted minimally-invasive surgery visualization device according to the present disclosure.
FIG. 3 is a use state diagram illustrating a first implementation of a fluid-assisted minimally-invasive surgery visualization device according to the present disclosure.
FIG. 4 is a structural sectional diagram illustrating a second implementation of a fluid-assisted minimally-invasive surgery visualization device according to the present disclosure.
FIG. 5 is a structural sectional diagram illustrating a third implementation of a fluid-assisted minimally-invasive surgery visualization device according to the present disclosure.
FIG. 6 is a partial structure diagram illustrating a third implementation of a fluid-assisted minimally-invasive surgery visualization device according to the present disclosure.
FIG. 7 is a structural sectional diagram illustrating a fourth implementation of a fluid-assisted minimally-invasive surgery visualization device according to the present disclosure.
FIG. 8 is a structural sectional diagram illustrating a fifth implementation of a fluid-assisted minimally-invasive surgery visualization device according to the present disclosure.
FIG. 9 is a structural sectional diagram illustrating a sixth implementation of a fluid-assisted minimally-invasive surgery visualization device according to the present disclosure.
FIG. 10 is a structural sectional diagram illustrating a seventh implementation of a fluid-assisted minimally-invasive surgery visualization device according to the present disclosure.
FIG. 11 is a structural sectional diagram illustrating an eighth implementation of a fluid-assisted minimally-invasive surgery visualization device according to the present disclosure.
FIG. 12 is a structural schematic diagram illustrating an inner pipe body according to the present disclosure.

### DETAILED DESCRIPTIONS OF EMBODIMENTS

The present disclosure will be set forth in details below in combination with the drawings.

### Embodiment 1

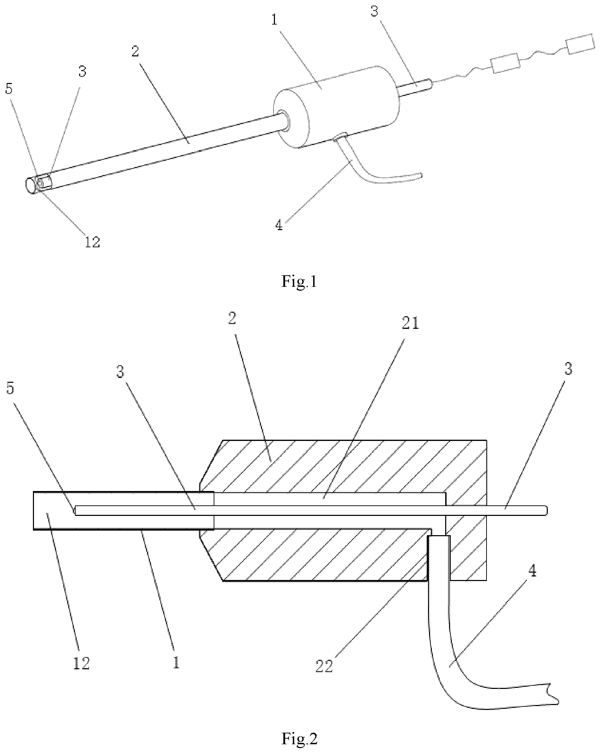

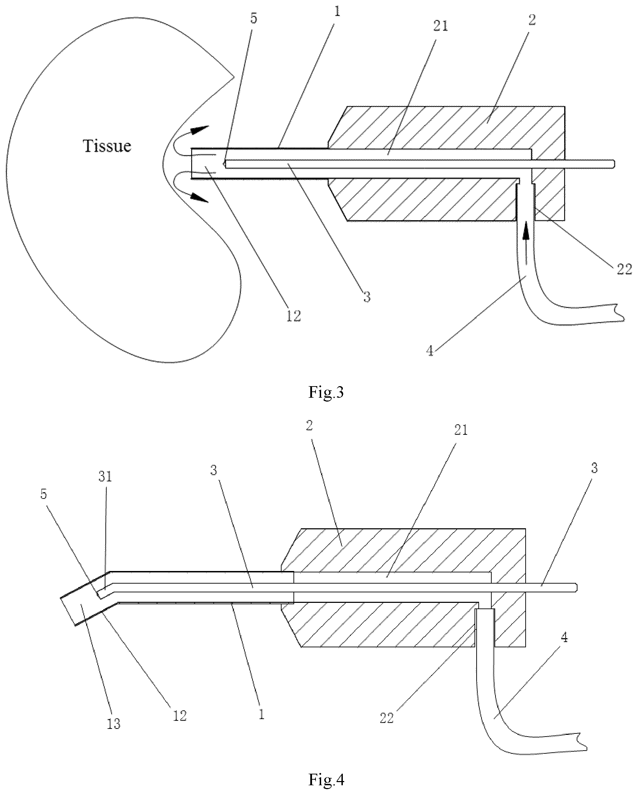

With reference to FIGS. 1 to 3, there is provided a fluid-assisted minimally-invasive surgery visualization device, including an outer sleeve assembly, an imaging module, an imaging module support body 3 and a water delivery pipe 4. The outer sleeve assembly includes an outer sleeve 1 and a sleeve seat 2. A water supply cavity 21 in communication with a rear end of the outer sleeve 1 is provided inside the sleeve seat 2. The water supply cavity 21 is further in communication with the water delivery pipe. The imaging module is supported on the outer sleeve assembly by the imaging module support body, and the imaging module is accommodated in the outer sleeve 1. One segment of clear water cavity 12 is formed between a front end surface of the outer sleeve 1 and a lens 5 of the imaging module. Specifically, the imaging module support body 3 is assembled inside the outer sleeve assembly and the imaging module is mounted inside the outer sleeve assembly by the imaging module support body 3. After the assembling is completed, the lens 5 of the imaging module is located inside the outer sleeve 1 and close to the front end surface of the outer sleeve 1. In this case, a small distance exists between the lens 5 of the imaging module and the front end surface of the outer sleeve 1 to define a cavity as the clear water cavity 12. In use, the lens 5 is located behind the front end surface of the outer sleeve 1, and clear normal saline is continuously drained from the front end of the outer sleeve 1. The clear water cavity 12 is formed between the front end surface of the outer sleeve 1 and the lens 5, and the normal saline in the water supply cavity 21 and the outer sleeve 1 has a particular water pressure which prevents the bloody water in a wound from entering the outer sleeve 1. Further, a local region at the front end of the outer sleeve 1 is a clear visual region which ensures a clear visual region is always maintained between the lens 5 and the front end of the outer sleeve 1, so as to help to clearly observe through the lens 5 the circumstances of the internal tissue of the wound and thus quickly find a site for surgical operation.

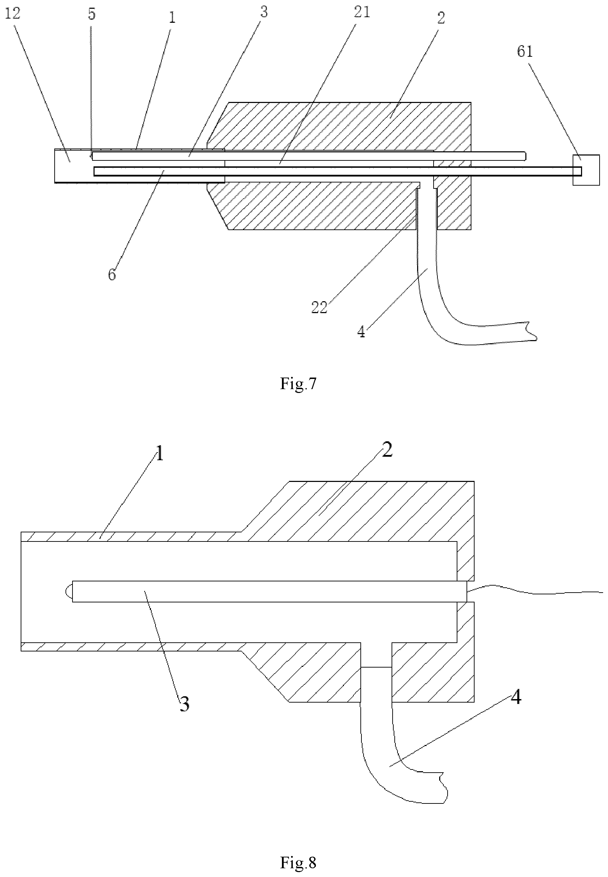

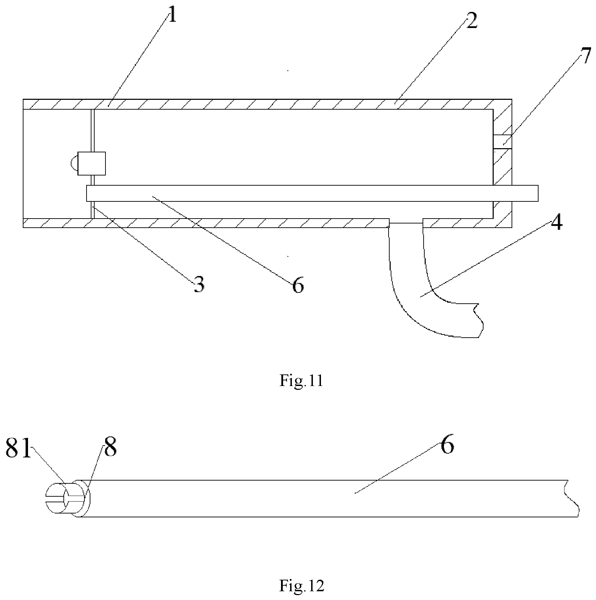

In one implementation, the outer sleeve 1 and the sleeve seat 2 are of one-piece structure, namely, the outer sleeve 1 and the sleeve seat 2 are integrally formed, as shown in FIG. 8. Preferably, the sleeve seat 2 has a larger diameter than the outer sleeve 1 to facilitate grip. Of course, the sleeve seat 2 may have the same diameter as the outer sleeve 1, namely, the outer sleeve 1 and the sleeve seat 2 are entirely formed into a thin-walled tubular structure, as shown in FIG. 11. Of course, an inner cavity of the sleeve seat 2 may be identical in shape and size to or different in shape and/or size from an inner cavity of the outer sleeve 1, which is not limited herein.

In another implementation, the outer sleeve 1 and the sleeve seat 2 are of split structure. As shown in FIG. 2, a rear end of the outer sleeve 1 is connected to a front end of the sleeve seat 2.

On the basis of the above two implementations of the outer sleeve 1 and the sleeve seat 2, the imaging module support body 3 may be supported on the sleeve seat 2 or supported on the outer sleeve 1. The imaging module support body may be disposed in many manners, for example, in the form of rod body, wire body, support bracket or a combination of rod body and support bracket or the like.

As shown in FIG. 2, the imaging module support body 3 is a rod body. Specifically, the rod body is assembled onto the sleeve seat 2, and the lens 5 of the imaging module is mounted at a front end of the rod body. Of course, the rod body may also be supported on the sleeve seat 2 or the outer sleeve 1 by a support bracket.

Furthermore, the rod body is disposed inside the outer sleeve 1 and supported at a rear part of the sleeve seat 2. Specifically, a first through hole is disposed at the rear part of the sleeve seat 2 and the rod body is mounted into the first through hole. An exit of the first through hole may be located at a rear end side of the sleeve seat 2 or at a side surface of non-end sides of the rear part of the sleeve seat 2. When the exit of the first through hole is located at the rear end side of the sleeve seat 2, the rear end part of the rod body may be protruded out of the rear end part of the sleeve seat 2 or be flushed with the rear end part of the sleeve seat 2 or be located inside the first through hole of the sleeve seat 2. Preferably, the rear end part of the rod body is located inside the first through hole of the sleeve seat 2, which, on the one hand, saves materials and reduces manufacturing costs, and on the other hand, helps grip the rear part of the sleeve seat 2. When the rear end part of the rod body is located inside the first through hole of the sleeve seat 2, if the lens is connected in a wired manner, a wire connected with the lens may be run through the first through hole. When the exit of the first through hole is located at a side surface of the non-end sides of the rear part of the sleeve seat 2, the rod body may be protruded out of the side surface of the sleeve seat 2 or be flushed with the side surface of the sleeve seat 2 or be located inside the first through hole of the sleeve seat 2. Preferably, when the rear end part of the rod body is located inside the first through hole of the sleeve seat 2, if the lens is connected in a wired manner, a wire connected with the lens may be run through the first through hole. Furthermore, when the lens is connected in a wireless manner, the rear part of the sleeve seat 2 may also not be provided with the first through hole. Thus, a blind hole may be disposed at the rear part of the sleeve seat 2 and the rod body is mounted into the blind hole, or the rod body is directly connected to an inner side surface of the rear part of the sleeve seat 2.

Preferably, the rod body is fixedly cooperated with the sleeve seat 2 in a sealing manner to ensure the sealing performance of the water supply cavity 21 and hence ensure stable water pressure inside the water supply cavity 21.

Preferably, the rod body is a round rod, and preferably, the rod body is a rigid straight rod with circular cross section. Preferably, the rod body is a hollow rod in which the wire connected with the lens is received. Furthermore, an axis of the rod body is parallel to an axis of the outer sleeve 1.

Furthermore, the lens 5 of the imaging module which is an existing imaging module in the prior arts is disposed at the front end of the rod body and has an illuminating function. The imaging module is connected with an external display device via a wire so as to display the circumstances of the internal tissue of the wound on the display device. Preferably, the lens 5 is embedded into the front end of the rod body and fixedly and sealingly fitted into the rod body to prevent the normal saline in the outer sleeve 1 from entering the rod body. When the lens 5 is connected in a wired manner, a wire connected with the lens 5 is received inside the rod body and run through the rear end of the rod body to connect with the external display device.

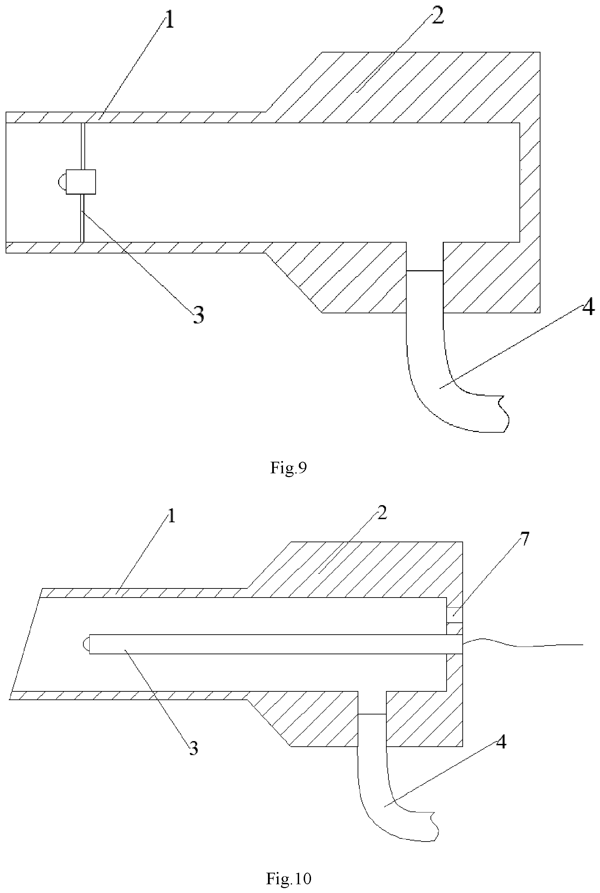

As shown in FIG. 9, the imaging module support body 3 is a support bracket, and the imaging module is supported on the outer sleeve by the support bracket. The structure of the support bracket may be a disk-type spoke shape (for example, shaped like a wheel spoke of a bicycle), a single rod shape (supported by single-spoke structure) or a symmetrical dual rod support (supported by two symmetrical spoke structures) or another structure capable of realizing the supporting, which is not limited herein.

Furthermore, the water delivery pipe 4 is disposed on the sleeve seat 2, one end of the water delivery pipe 4 is in communication with the outer sleeve 1 through the water supply cavity 21 and the other end is connected to a water supply device. Specifically, a water supply hole 22 in communication with the water supply cavity 21 is disposed on a wall of the sleeve seat 2. The water supply hole may be located on a sidewall of the sleeve seat 2 or on a rear end wall of the sleeve seat 2. The water delivery pipe 4 is in communication with the water supply hole, which specifically means that: one end of the water delivery pipe 4 is inserted into the water supply hole 22 while an outer sidewall of the water delivery pipe 4 is fixedly and sealingly fitted to an inner wall of the water supply hole 22. The water supply device is in communication with the interior of the outer sleeve 1 through the water delivery pipe 4. During a surgical operation process, clear normal saline is continuously input into the outer sleeve 1 and then into the interior of the wound through the front end of the outer sleeve 1.

Furthermore, the front end surface of the outer sleeve is an oblique surface as shown in FIG. 10. The disposal of the oblique surface structure helps the outer sleeve to pierce into the interior of the wound operated for the patient.

When the outer sleeve 1 and the sleeve seat 2 are of split structure:
Furthermore, the outer sleeve 1 is a tubular structure with openings at both ends. Preferably, the outer sleeve is a circular straight pipe, more preferably, the outer sleeve is a rigid circular straight pipe. Of course, the outer sleeve may also be a stepped pipe or a bent pipe with its sectional shape being a non-circle, for example, quadrangle, hexagon, octagon or the like, which is not limited herein.

Furthermore, the outer sleeve 1 may be directly fixed at the front end of the sleeve seat 2 or may be detachably connected to the front end of the sleeve seat 2. For example, the outer sleeve 1 may be connected to the front end of the sleeve seat 2 by insertion connection, snap fit connection or thread connection. The connection of the outer sleeve 1 by insertion connection, snap fit connection or thread connection may, on the one hand, facilitate processing and assembling, and on the other hand, help change to an outer sleeve of a different length and a different hole diameter.

Furthermore, the water supply cavity 21 is a cavity with a front end opened and a rear end closed, namely, the front end of the water supply cavity 21 is provided with an water outlet consistent in shape and size with a longitudinal section of the water supply cavity 21 or not consistent in shape and size with the longitudinal section of the water supply cavity 21. For example, the water outlet is smaller than the longitudinal section of the water supply cavity 21 or the like. The drawing illustrates that the water outlet is consistent in size and shape with the longitudinal section of the water supply cavity 21.

Preferably, the rear end of the outer sleeve 1 is inserted into the front end of the water supply cavity 21 and hence fixedly and sealingly connected with the sleeve seat 2. Under a working state, when the outer sleeve 1 is inserted into the interior of the wound operated for the patient, the sleeve seat 2 is gripped by hand to adjust a depth of the outer sleeve 1 into the wound so as to find a site to be operated.

In a preferred embodiment, all or part of the outer sleeve 1 is flexible, that is, all or part of the outer sleeve 1 is a flexible pipe. Correspondingly, if the imaging module support body is a rod body, all or part of the rod body is flexible; if the imaging module support body is a wire body, all or part of the wire body is flexible. With all or part of the outer sleeve and the imaging module support body disposed as flexible, the surgical instruments satisfy the flexibility requirements, leading to wider application scope.

In this embodiment, the lens of the imaging module is located inside the outer sleeve, and one segment of clear water is maintained between the lens and the front end surface of the outer sleeve so as to form a clear water cavity. Clear water flowing forward inside the outer sleeve forms a clear visual region. When the front end of the outer sleeve approaches a tissue surface, the clear water inside it flushes away the bloody water to make the tissue surface clearly observable, thereby improving the accuracy and efficiency of the surgical operation and eliminating the possibility of mis-operation.

### Embodiment 2

The embodiment 2 is further improved on the basis of the embodiment 1. In combination with FIGS. 1 to 4, the technical solution of the embodiment 2 has substantially the same contents and working principle as the technical solution of the embodiment 1 except for the following differences: the front end part of the outer sleeve 1 is bent aside to form a first bending portion 13 which is bent aside 5 to 20° preferably 15°, relative to the main body of the outer sleeve 1; the front end part of the imaging module support body 3 is bent aside to form a second bending portion 31 which is bent aside relative to the main body of the imaging module support body 3 at the same angle as the first bending portion bent aside relative to the main body of the outer sleeve 1; the second bending portion 31 is consistent in direction with the first bending portion 13; and the clear water cavity 12 is located inside the first bending portion. The disposal of the bending portions can expand an observation region of a tissue. Specifically, under a working state, when the outer sleeve 1 is inserted into an interior of a wound operated for a patient, the sleeve seat 2 is gripped by hand to adjust the depth of the outer sleeve 1 into the wound while the observation region of the tissue can be expanded by rotation of the sleeve seat 2.

### Embodiment 3

The embodiment 3 is further improved on the basis of the embodiment 1 or 2. In combination with FIGS. 1, 2, 3, 5 and 6, the technical solution of the embodiment 3 has substantially the same contents and working principle as the technical solution of the embodiment 1 except for the following differences: the front end of the outer sleeve 1 is provided with a connection portion used for connection with an operation tool. The operation tool may be detachably connected to the front end of the outer sleeve 1 by the connection portion, where the connection portion may be a snap fit portion, a thread connection portion or an insertion portion.

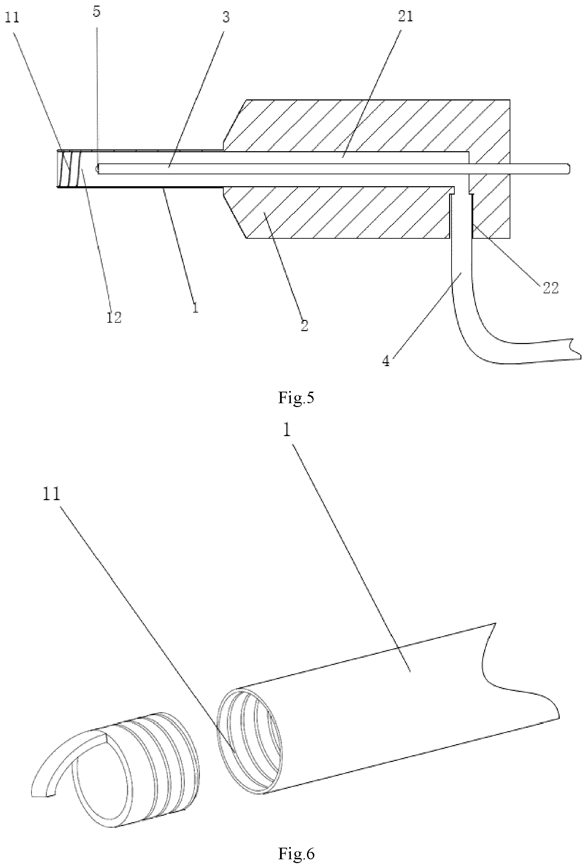

Preferably, internal threads for connecting the operation tool are disposed on an inner sidewall of the front end of the outer sleeve 1 and further located ahead of the lens 5, as shown in FIG. 5. The operation tool may be detachably and fixedly mounted to the front end of the outer sleeve 1 by the internal threads 11. Under visual conditions, operation is performed on a site for surgical operation found on the tissue to realize integration of lens and instrument, thereby improving the operation accuracy and efficiency and greatly reducing the occurrence of mis-operations.

### Embodiment 4

The embodiment 4 is further improved on the basis of the embodiment 1. In combination with FIGS. 1, 2 and 3, the fluid-assisted minimally-invasive surgery visualization device includes an outer sleeve assembly, an imaging module, an imaging module support body 3 and a water delivery pipe 4. The outer sleeve assembly includes an outer sleeve 1 and a sleeve seat 2. Further, one or more operation holes 7 are disposed on the rear end of the sleeve seat 2, and the operation hole 7 forms a working channel for communicating the interior of the outer sleeve assembly with the outside. Preferably, the operation hole 7 is further provided with a sealing cap 61.

Specifically, during a working process, when a tissue lesion or bleeding position is searched for, the sealing cap 7 can seal the operation hole 7 and after an operation position of a wound tissue is determined by using the lens 5 under clear visual conditions, the sealing cap is opened. Thus, a minimally-invasive operation tool, for example, a minimally invasive forceps or a minimally-invasive scalpel may penetrate through the operation hole 7 and then through the outer sleeve assembly into a surgery site for surgical operation. The clear water cavity at the front end of the outer sleeve 1 can provide clear observation conditions for surgical operation, thus improving the surgical efficiency and accuracy and reducing surgical mis-operations.

The operation hole may be in the shape of circle, square, rectangle, hexagon, octagon and ellipse and the like. When there are multiple operation holes, the shapes of the operation holes may be same or different.

Furthermore, as shown in FIG. 7, the device further includes an inner pipe body 6 disposed at the operation hole 7. A working channel for communicating the interior of the outer sleeve 1 with the outside is formed inside the inner pipe body 6, and the inner pipe body 6 and the imaging module support body 3 are disposed in parallel.

Preferably, the front end surface of the inner pipe body 6 is located behind the lens 5, and preferably, the inner pipe body 6 is a straight round pipe with equal section.

The number of the inner pipe bodies 6 may be equal to or unequal to the number of the operation holes 7.

Specifically, the inner pipe body 6 is supported on the operation hole 7; an exit of the operation hole 7 is located at a rear end side of the sleeve seat 2 and the rear end part of the inner pipe body 6 protrude out of the rear end side of the sleeve seat 2 or flush with the rear end side of the sleeve seat 2 or located in the operation hole 7 of the sleeve seat 2. Preferably, the rear end of inner pipe body 6 runs out of the sleeve seat 2 and accordingly, the sealing cap 61 is disposed at the rear end of the inner pipe body 6. During operation process, a tissue lesion or bleeding position is searched for, the sealing cap 61 seals the rear end of the inner pipe body 6, and after an operation position of a wound tissue is determined by using the lens 5 under clear visual conditions, the sealing cap is opened. Thus, a minimally-invasive operation tool, for example, a minimally invasive forceps or a minimally-invasive scalpel may penetrate through the inner pipe body 6 into a surgery site for surgical operation. The clear water cavity at the front end of the outer sleeve 1 can provide clear observation conditions for surgical operation, thus improving the surgical efficiency and accuracy and reducing surgical mis-operations.

Furthermore, as shown in FIG. 12, a front end of the inner pipe body 6 is provided with an elastic tightening portion 8 with an inner diameter less than an inner diameter of the inner pipe body 6. The elastic tightening portion 8 is used to tightly clamp the minimally-invasive surgery tool. Since the diameter or thickness of the minimally-invasive surgery tool is generally smaller than the inner diameter of the inner pipe body 6, when the minimally-invasive surgery tool is inserted into the inner pipe body for surgical operation, there is a gap between the minimally-invasive surgery tool and the inner wall of the inner pipe body, leading to unstable operation of the minimally-invasive surgery tool during a surgical operation process. But, the elastic tightening portion may be disposed at an end of the inner pipe body close to the lens to clamp the minimally-invasive surgery tool, so as to ensure stable operation of the minimally-invasive surgery tool during the surgical operation process.

Specifically, the elastic tightening portion 8 is formed by enclosing at least two clamping sheets 81. The elastic tightening portion 8 formed by multiple clamping sheets can tightly clamp the minimally-invasive surgery tool under the elastic action of the clamping sheets. Therefore, it is structurally simple and easy to manufacture. Of course, the elastic tightening portion may also be another structure, for example, an elastic pad is attached to an inner wall of the front end of the inner pipe body, or an elastic clip is attached to the inner wall of the front end of the inner pipe body or the like, so as to implement the structure of the minimally-invasive surgery tool.

It is to be noted that in the present disclosure, the structural disposal of the outer sleeve assembly (for example, integral structure, or split structure, thickness and shape and the like), the structure of the imaging module support body (for example, rod body, hard wire body, or support bracket or the like), support position (outer sleeve or sleeve seat), the disposal or non-disposal of the operation hole and the number and shape of the operation holes, and the disposal or non-disposal of the inner pipe body and the number and shape of the inner pipe bodies can be combined arbitrarily but not limited to the implementations shown in the drawings.

Those parts not mentioned in the present disclosure can be implemented by using the prior arts. In the descriptions of the present disclosure, it is understood that orientation or positional relationship indicated by the terms such as "central", "longitudinal", "transverse", "upper", "lower", "front", "rear", "left", "right", "vertical", "horizontal", "top", "bottom", "inside", "outside", and the like is used only for ease of descriptions and simplification of descriptions and does not indicate or imply that the indicated devices or elements must have a particular orientation, or be constructed or operated in a particular orientation. Therefore, such terms shall not be understood as limiting of the present disclosure.

Further, the terms "first" and "second" are used for descriptions only and shall not be understood as indicating or implying relative importance. Of course, the above descriptions are not intended to limit the present disclosure and the present disclosure is also not limited to the above examples. Changes, modifications, additions or replacements made by those skilled in the arts within the essence scope of the present disclosure shall all fall within the scope of protection of the present disclosure.

## Claims

1. A fluid-assisted minimally-invasive surgery visualization device, comprising an outer sleeve assembly, an imaging module, an imaging module support body and a water delivery pipe; wherein the outer sleeve assembly comprises an outer sleeve and a sleeve seat, a water supply cavity in communication with a rear end of the outer sleeve is provided inside the sleeve seat, and the water supply cavity is further in communication with the water delivery pipe; the imaging module is supported on the outer sleeve assembly by the imaging module support body, the imaging module is accommodated in the outer sleeve, and one segment of clear water cavity is formed between a front end surface of the outer sleeve and a lens of the imaging module.

2. The fluid-assisted minimally-invasive surgery visualization device of claim 1, wherein the outer sleeve and the sleeve seat are of integral structure or of split structure.

3. The fluid-assisted minimally-invasive surgery visualization device of claim 2, wherein the imaging module support body is supported on the outer sleeve or on the sleeve seat.

4. The fluid-assisted minimally-invasive surgery visualization device of claim 3, wherein the imaging module support body is a rod body, or a support bracket, or a hard wire body, or a cooperative structure of rod body and support bracket.

5. The fluid-assisted minimally-invasive surgery visualization device of claim 4, wherein the imaging module support body is a rod body, a rear end of the rod body is supported on a rear part of the sleeve seat, a first through hole is disposed at a part of the sleeve seat located behind the water supply cavity, and the rod body is supported and mounted at the rear part of the sleeve seat by the first through hole.

6. The fluid-assisted minimally-invasive surgery visualization device of claims 1 to 5, wherein all or part of the outer sleeve is a flexible pipe.

7. The fluid-assisted minimally-invasive surgery visualization device of any one of claims 1 to 5, wherein a water supply hole in communication with the water supply cavity is disposed on a wall of the sleeve seat; and the water supply hole is connected with the water delivery pipe.

8. The fluid-assisted minimally-invasive surgery visualization device of any one of claims 1 to 5, wherein a front end part of the outer sleeve is bent aside to form a first bending portion, a front end part of the imaging module support body is bent aside to form a second bending portion, the second bending portion is consistent in direction with the first bending portion, and the clear water cavity is located inside the first bending portion.

9. The fluid-assisted minimally-invasive surgery visualization device of any one of claims 1 to 5, wherein the front end of the outer sleeve is provided with a connection portion used for connecting an operation tool.

10. The fluid-assisted minimally-invasive surgery visualization device of claim 9, wherein the connection portion is internal threads for connecting the operation tool, which are disposed on an inner sidewall of the front end of the outer sleeve, and located ahead of the lens.

11. The fluid-assisted minimally-invasive surgery visualization device of any one of claims 1 to 5, wherein the rear end of the sleeve seat is further provided with one or more operation holes, and the operation hole forms a working channel communicating the interior of the outer sleeve assembly with the outside.

12. The fluid-assisted minimally-invasive surgery visualization device of claim 11, further comprising an inner pipe body, wherein the inner pipe body is disposed at the operation hole, a working channel communicating the interior of the outer sleeve with the outside is formed inside the inner pipe body, and the number of the inner pipe bodies is equal to or less than the number of the operation holes.

13. The fluid-assisted minimally-invasive surgery visualization device of claim 12, wherein a front end surface of the inner pipe body is located behind the lens.

14. The fluid-assisted minimally-invasive surgery visualization device of claim 12 or 13, wherein a front end of the inner pipe body is provided with an elastic tightening portion with an inner diameter less than an inner diameter of the inner pipe body.

15. The fluid-assisted minimally-invasive surgery visualization device of claim 11, further comprising a sealing cap for sealing the operation hole.
